# EUROPEAN PATENT SPECIFICATION

(11) **EP 2 996 486 B1**
(45) Date of publication and mention of the grant of the patent: **23.10.2019**
(21) Application number: 13884677.9
(22) Date of filing: 17.05.2013
(51) Int. Cl.: A23G 3/00, A23L 27/00

(54) **PROCESS FOR OBTAINING A STEVIA COMPOSITION**
VERFAHREN ZUR HERSTELLUNG EINER STEVIA-ZUSAMMENSETZUNG
PROCEDE DE PRODUCTION D'UNE COMPOSITION DE STEVIA

(30) Priority: 15.05.2013 US 201313885591
(43) Date of publication of application: 23.03.2016
(73) Proprietor: Purecircle Usa Inc., Oak Brook, IL 60523-1492 (US)
(72) Inventor: MARKOSYAN, Avetik, 0014 Yerevan (AM)
(74) Representative: Vossius & Partner Patentanwälte Rechtsanwälte mbB
(86) International application number: PCT/US2013/041609
(87) International publication number: WO 2014/185931

(56) References cited:
- WO-A1-2012/082493
- WO-A1-2012/166164
- WO-A1-2012/166164
- WO-A1-2013/036366
- WO-A1-2013/058870
- WO-A2-2008/091547
- US-A- 4 219 571
- US-A- 4 454 290
- US-A- 4 590 160
- US-A- 4 599 403
- US-A1- 2007 116 819
- US-A1- 2009 074 935
- US-A1- 2010 278 993
- US-A1- 2010 278 993
- US-A1- 2011 092 684
- US-B1- 6 204 377
- SERGEI VICTROVICH LOBOV ET AL: "Enzymic Production of Sweet Stevioside Derivatives: Transglucosylation by Glucosidases.", AGRICULTURAL AND BIOLOGICAL CHEMISTRY, vol. 55, no. 12, 1 December 1991 (1991-12-01), pages 2959-2965, XP55125445, ISSN: 0002-1369, DOI: 10.1271/bbb1961.55.2959

## Description

### BACKGROUND OF THE INVENTION

### Field Of The Invention

The invention is defined by the claims. It relates to a process for producing a stevia composition comprising rebaudioside B as defined by the claims.

### Description Of The Related Art

Sugar alternatives are receiving increasing attention due to the awareness of many diseases associated with the consumption of high-sugar foods and beverages. However, many artificial sweeteners such as dulcin, sodium cyclamate and saccharin have been banned or restricted in some countries due to concerns about their safety. As a result, non-caloric sweeteners of natural origin are becoming increasingly popular. The sweet herb *Stevia rebaudiana* Bertoni produces a number of diterpene glycosides which feature high intensity sweetness and sensory properties superior to those of many other high potency sweeteners.

The above-mentioned sweet glycosides, have a common aglycon, steviol, and differ by the number and type of carbohydrate residues at the C13 and C19 positions. The leaves of Stevia are able to accumulate up to 10-20% (on dry weight basis) steviol glycosides. The major glycosides found in Stevia leaves are rebaudioside A (2-10%), stevioside (2-10%), and rebaudioside C (1-2%). Other glycosides such as rebaudioside *B, D, E,* and *F*, steviolbioside and rubusoside are found at much lower levels (approx. 0-0.2%).

Two major glycosides - stevioside and rebaudioside *A* (reb *A*)*,* were extensively studied and characterized in terms of their suitability as commercial high intensity sweeteners. Stability studies in carbonated beverages confirmed their heat and pH stability (Chang S.S., Cook, J.M. (1983) Stability studies of stevioside and rebaudioside A in carbonated beverages. J. Agric. Food Chem. 31: 409-412.)

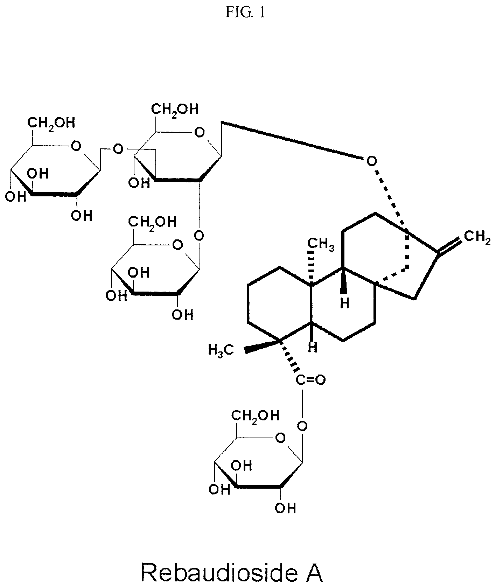

Steviol glycosides differ from each other not only in their molecular structures, but also by their taste properties. Usually stevioside is found to be 110-270 times sweeter than sucrose, rebaudioside *A* between 150 and 320 times sweeter than sucrose,, and rebaudioside *C* between 40-60 times sweeter than sucrose. Dulcoside *A* is 30 times sweeter than sucrose. Rebaudioside *A* has the least astringent, the least bitter, and the least persistent aftertaste, thus possessing the most favorable sensory attributes in major steviol glycosides (Tanaka O. (1987) Improvement of taste of natural sweetners. Pure Appl. Chem. 69:675-683; Phillips K.C. (1989) Stevia: steps in developing a new sweetener. In: Grenby T.H. ed. Developments in sweeteners, vol. 3. Elsevier Applied Science, London. 1-43.) The chemical structure of rebaudioside *A* is shown in Fig. 1.

Methods for the extraction and purification of sweet glycosides from the *Stevia rebaudiana* plant using water or organic solvents are described in, for example, U.S. Patent Numbers 4,361,697; 4,082,858; 4,892,938; 5,972,120; 5,962,678; 7,838,044 and 7,862,845.

However, even in a highly purified state, steviol glycosides still possess undesirable taste attributes such as bitterness, sweet aftertaste, licorice flavor, etc. One of the main obstacles for the successful commercialization of stevia sweeteners are these undesirable taste attributes. It was shown that these flavor notes become more prominent as the concentration of steviol glycosides increases (Prakash I., DuBois G.E., Clos J.F., Wilkens K.L., Fosdick L.E. (2008) Development of rebiana, a natural, non-caloric sweetener. Food Chem. Toxicol., 46, S75-S82.).

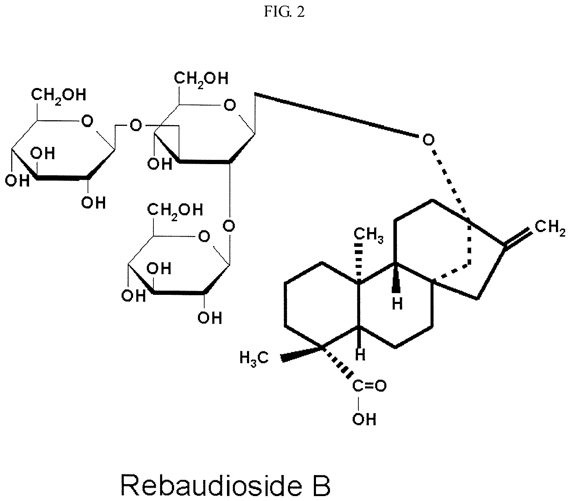

Rebaudioside *B* (CAS No: 58543-17-2), or reb *B,* also known as stevioside *A₄* (Kennelly E.J. (2002) Constituents of Stevia rebaudiana In Stevia: The genus Stevia, Kinghorn A.D. (Ed), Taylor & Francis, London, p.71), is one of the sweet glycosides found in *Stevia rebaudiana.* Sensory evaluations show that reb *B* was approximately 300-350 times sweeter than sucrose, while for reb *A* this value was approximately 350-450 (Crammer, B. and Ikan, R. (1986) Sweet glycosides from the Stevia plant. Chemistry in Britain 22, 915-916, and 918). The chemical structure of rebaudioside *B* is shown in Fig. 2.

It was believed that reb *B* forms from the partial hydrolysis of rebaudioside *A* during the extraction process (Kobayashi, M., Horikawa, S., Degrandi, I.H., Ueno, J. and Mitsuhashi, H. (1977) Dulcosides A and B, new diterpene glycosides from Stevia rebaudiana. Phytochemistry 16, 1405-1408). However, further research has shown that reb *B* occurs naturally in the leaves of *Stevia rebaudiana* and is currently one of nine steviol glycosides recognized by FAO/JECFA (United Nations' Food and Agriculture Organization/Joint Expert Committee on Food Additives) in calculating total steviol glycosides' content in commercial steviol glycoside preparations (FAO JECFA (2010) Steviol Glycosides, Compendium of Food Additive Specifications, FAO JECFA Monographs 10, 17-21).

Only a few methods are described in literature for preparing reb *B.*

Kohda et al., (1976) prepared reb *B* by hydrolysis of reb *A* with hesperidinase. Reb *B* was also prepared by alkaline saponification of reb *A.* The said saponification was conducted in 10% potassium hydroxide-ethanol. The solution was acidified with acetic acid, and extracted with n-butanol. The butanol layer was washed with water and concentrated at low temperature *in vacuo.* The residue was crystallized from methanol to give reb *B.* (Kohda, H., Kasai, R., Yamasaki, K., Murakami, K. and Tanaka, O. (1976) New sweet diterpene glucosides from Stevia rebaudiana. Phytochemistry 15, 981-983). The described processes might be suitable for laboratory scale preparation of reb *B,* but are not suitable for any large scale or commercial reb *B* preparation. As disclosed in WO 2012/166164, stevia compositions comprising reb B may be prepared from compositions comprising reb A using biocatalysts capable of hydrolysing beta-glucosyl ester bonds, among which hesperidinase and beta-glucosidases are disclosed.

Ahmed et al., used mild alkaline hydrolysis of reb *A* to prepare reb *B.* According to the described procedure, reb *A* was hydrolyzed to reb *B* by refluxing with 10% aqueous KOH at 100°C for 1hr. After neutralization with glacial acetic acid, the precipitated substance was recrystallized twice from methanol (Ahmed M.S., Dobberstein R.H., and Farnsworth N.R. (1980) Stevia rebaudiana: I. Use of p-bromophenacyl bromide to enhance ultraviolet detection of water-soluble organic acids (steviolbioside and rebaudioside B) in high-performance liquid chromatographic analysis, J. Chromatogr., 192, 387-393).

The use of methanol as recrystallization media as described in the literature will require its subsequent removal from the product. It is noted that handling of toxic substances such as methanol requires specialized manufacturing installations and, when applied in food processing, sophisticated food safety measures.

It is also noted that no significant work has been conducted to determine the potential of reb *B* as a sweetener or food ingredient. Moreover, reb *B* is often viewed as process artifact and unnecessary impurity in commercial steviol glycosides preparations. No significant evaluation of the influence of reb *B* on the overall taste profile of steviol glycoside preparations has been conducted.

The water solubility of reb *B* is reported to be about 0.1% (Kinghorn A.D. (2002) Constituents of Stevia rebaudiana In Stevia: The genus Stevia, Kinghorn A.D. (Ed), Taylor & Francis, London, p.8). In many food processes where highly concentrated ingredients are used, a highly soluble form of reb *B* will be necessary.

Considering the facts mentioned above, there is a need to evaluate reb *B* as a sweetener and food ingredient and to develop a simple and efficient process for food grade reb *B* preparations suitable for food and other applications.

Within the description of this invention we will show that, when applied in specific manner, reb *B* may impact the taste profile and offer significant advantages for the use of stevia sweeteners in various applications.

### SUMMARY

The present invention is defined by the claims. Accordingly, the present invention relates to a process for producing a stevia composition comprising rebaudioside *B*, comprising the steps of: providing a stevia sweetener comprising rebaudioside *A*; providing a biocatalyst capable of hydrolyzing *β*-glucosyl ester bonds in the stevia sweetener, wherein the biocatalyst is lactase; dissolving the stevia sweetener and adding the biocatalyst to make a reaction mixture; incubating the reaction mixture for about 12 to 48 hours to at least partially hydrolyze *β*-glucosyl ester bonds in the stevia sweetener; cooling the mixture to about 10-30°C and adjusting the pH with acid to about pH 3.0-4.0; incubating the mixture at low temperature to obtain a precipitate; separating the precipitate and washing the precipitate with water; and drying the washed precipitate to obtain the stevia composition. The invention is aimed to overcome the disadvantages of existing Stevia sweeteners. Also described herein is a process for producing a high purity food ingredient from the extract of the *Stevia rebaudiana* Bertoni plant and use thereof in various food products and beverages as a sweetness and flavor modifier.

The disclosure, in part, pertains to an ingredient comprising steviol glycosides of *Stevia rebaudiana* Bertoni plant. The steviol glycodsides are selected from the group consisting of stevioside, rebaudioside *A* (FIG. 1), rebaudioside *B* (FIG. 2), rebaudioside *C*, rebaudioside *D,* rebaudioside *E*, rebaudioside *F*, dulcoside *A*, steviolbioside, rubusoside, as well as other steviol glycosides found in *Stevia rebaudiana* Bertoni plant and mixtures thereof.

The disclosure, in part, pertains to a process for producing an ingredient containing rebaudioside *B,* and stevioside, rebaudioside *A,* rebaudioside *C*, rebaudioside *D,* rebaudioside *E*, rebaudioside *F*, dulcoside *A*, steviolbioside, rubusoside, as well as other steviol glycosides found in *Stevia rebaudiana* Bertoni plant and mixtures thereof.

Herein, rebaudioside *A* commercialized by PureCircle Sdn. Bhd. (Malaysia), containing, rebaudioside *A* (about 95-100%), stevioside (about 0-1%), rebaudioside *C* (about 0-1%), rebaudioside *F* (about 0-1%), rebaudioside *B* (about 0.1-0.8%), rebaudioside *D* (about 0-1%), and other glycosides amounting to total steviol glycosides' content of at least 95%, may be used as a starting material. Alternatively stevia extracts with different ratios of steviol glycosides may be used as starting materials.

The starting material is subjected to complete or partial conversion into reb *B* using a biocatalyst capable of hydrolyzing *β*-glucosyl ester bonds. The obtained glycoside mixtures can be used "as-is" as well as by recovering reb *B* from the mixture and using it as a pure ingredient.

The low solubility reb *B* may be subjected to additional thermal treatment to increase solubility.

The obtained products were applied in various foods and beverages as sweeteners, sweetener enhancers and flavor modifiers, including soft drinks, ice cream, cookies, bread, fruit juices, milk products, baked goods and confectionary products.

It is to be understood that both the foregoing general description and the following detailed description are explanatory and are intended to provide further explanation of the invention as claimed.

### BRIEF DESCRIPTION OF THE DRAWINGS

The accompanying drawings are included to provide a further understanding of the invention. The drawings illustrate embodiments of the invention and together with the description serve to explain the principles of the embodiments of the invention.
FIG. 1 shows the chemical structure of rebaudioside *A.*
FIG. 2 shows the chemical structure of rebaudioside *B*
FIG. 3 shows an HPLC chromatogram of a stevia composition comprising rebaudioside *A* and rebaudioside *B.*

### DETAILED DESCRIPTION

Advantages of the present invention will become more apparent from the detailed description given hereinafter.

Rebaudioside *A* commercialized by PureCircle Sdn. Bhd. (Malaysia), containing, rebaudioside *A* (about 95-100%), stevioside (about 0-1%), rebaudioside *C* (about 0-1%), rebaudioside *D* (about 0-1%), rebaudioside *F* (about 0-1%), rebaudioside *B* (about 0.1-0.8%) and other glycosides amounting to total steviol glycosides' content of at least about 95%, may be used as a starting material. Alternatively stevia extracts with different ratios of steviol glycosides may be used as starting materials.

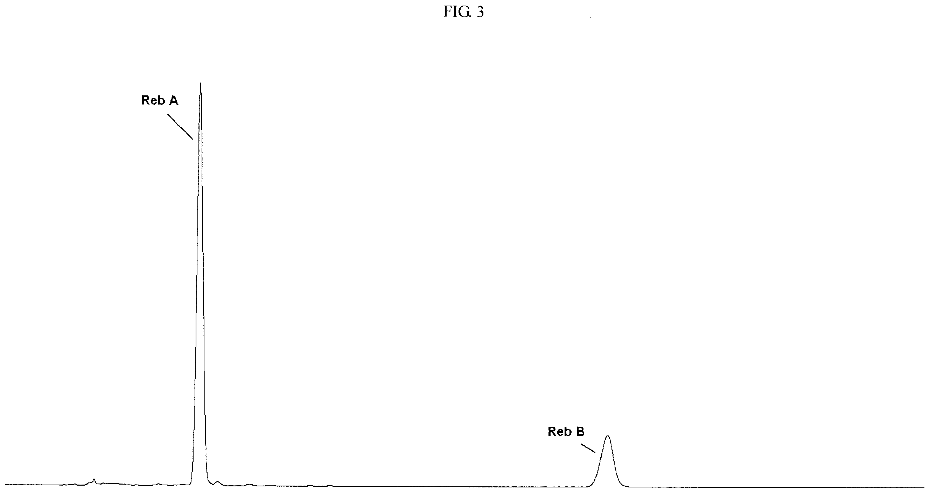

The HPLC analysis of the raw materials and products can be performed on an Agilent Technologies 1200 Series (USA) liquid chromatograph, equipped with Phenomenex Prodigy ODS3, 5 µm (4.6X250mm) column at 40°C. The mobile phase was 32:68 mixture of acetonitrile and 10 mmol/L sodium phosphate buffer (about pH 2.6) at 1 mL/min. A diode array detector set at 210 nm can be used as the detector. One example of an HPLC chromatogram thus obtained is shown in FIG. 3.

As used herein, unless specified further, "reb *B"* and "reb *B* composition" shall be used interchangeably to refer to purified rebaudioside *B* or rebaudioside *B* in combination with any other chemical entity.

### Preparation of Reb B

### 1. Biocatalytic Conversion

In one aspect described herein, reb *A* is dispersed in water to form solution. The concentration of reb *A* is about 0-50% (w/v) preferably about 10-25%. An enzyme preparation selected from group of esterases, lipases, cellulases, hemicellulases, hesperidinases, lactases and *β*-glucosidases, or any enzyme capable of hydrolyzing *β*-glucosyl ester bonds, or free or immobilized cells, or any other biocatalysts capable of hydrolyzing *β*-glucosyl ester bonds (the enzyme preparations, enzymes, free or immobilized cells, and other biocatalysts hereinafter collectively referred to as "biocatalysts") are added to reb *A* solution to form the reaction mixture. The mixture is incubated at about 10-150°C, preferably about 30-100°C, for a period of about 0.5-72hrs, preferably about 1-48 hrs. As a result reb *A* is hydrolyzed to reb *B.* The molar yield of conversion of reb *B* is about 5-100%, preferably about 90-100%.

After the reaction, the biocatalyst is inactivated by heating or removal from the reaction mixture. The pH of obtained mixture is adjusted by an acid, preferably by sulfuric acid or ortho-phosphoric acid, until a pH of about 3.0-5.0 is reached, preferably until a pH of about 3.0-4.0 is reached. Upon acidification, a precipitate is formed. The precipitate is separated by any method known in the art such as filtration or centrifugation and washed with water until the water reaches a pH of about 4.0-5.0. The obtained crystalline material is dried under vacuum at about 60-105°C to yield a mixture of reb *A* and reb *B* having a ratio of about 1%:99% to about 99%:1% (w/w), preferably about 5%:95% to about 1%:99% (w/w).

### 2. Optional Post-Conversion Purification

To obtain purified reb *B*, the separated precipitate described above may be suspended in water and the mixture is subjected to continuous agitation over about 0.5-24 hrs, preferably about 1-3 hours, at about 50-100°C, preferably about 60-80°C. The ratio of precipitate to water (w/v) is about 1:5 to about 1:20, preferably about 1:10 to about 1:15. The washed crystals are separated and dried under vacuum at about 60-105°C to yield reb *B* with about 99% purity.

### 3. Optional Post-Conversion Solubility Enhancement

The following procedure can be used to increase the water solubility of reb *B* or any reb B composition. The obtained compositions generally have a water solubility of less than about 0.2% (w/v). In order to increase the solubility of these compositions, the compositions were combined with the water at ratio of about1:1 (w/w) and the obtained mixture was further subjected to a gradient heat treatment which resulted in a high stability and high concentration solution. The gradient of about 1°C per minute was used in heating the mixture. The mixture was heated to the temperature of about 110-140°C, preferably about 118-125°C and was held at maximum temperature for about 0-120 min, preferably about 50-70 min. After the heat treatment, the solution was cooled down to room temperature at gradient of about 1°C per minute. The solution was spray dried by a laboratory spray drier operating at about 175°C inlet and about 100°C outlet temperatures. An amorphous form of the composition was obtained with greater than about 20% solubility in water at room temperature.

### Use of Reb B Compositions

The reb *B* compositions described above can be used as a sweetness enhancer, a flavor enhancer and/or a sweetener in various food and beverage products. Non-limiting examples of food and beverage products include carbonated soft drinks, ready to drink beverages, energy drinks, isotonic drinks, low-calorie drinks, zero-calorie drinks, sports drinks, teas, fruit and vegetable juices, juice drinks, dairy drinks, yoghurt drinks, alcohol beverages, powdered beverages, bakery products, cookies, biscuits, baking mixes, cereals, confectioneries, candies, toffees, chewing gum, dairy products, flavored milk, yoghurts, flavored yoghurts, cultured milk, soy sauce and other soy base products, salad dressings, mayonnaise, vinegar, frozen-desserts, meat products, fish-meat products, bottled and canned foods, tabletop sweeteners, fruits and vegetables.

Additionally the compositions can be used in drug or pharmaceutical preparations and cosmetics, including but not limited to toothpaste, mouthwash, cough syrup, chewable tablets, lozenges, vitamin preparations, and the like.

The compositions can be used "as-is" or in combination with other sweeteners, flavors and food ingredients.

Non-limiting examples of sweeteners include steviol glycosides, stevioside, rebaudioside *A,* rebaudioside *B,* rebaudioside *C*, rebaudioside *D,* rebaudioside *E*, rebaudioside *F*, dulcoside *A,* steviolbioside, rubusoside, as well as other steviol glycosides found in *Stevia rebaudiana* Bertoni plant and mixtures thereof, stevia extract, Luo Han Guo extract, mogrosides, high-fructose corn syrup, corn syrup, invert sugar, fructooligosaccharides, inulin, inulooligosaccharides, coupling sugar, maltooligosaccharides, maltodextins, corn syrup solids, glucose, maltose, sucrose, lactose, aspartame, saccharin, sucralose, sugar alcohols.

Non-limiting examples of flavors include lemon, orange, fruit, banana, grape, pear, pineapple, bitter almond, cola, cinnamon, sugar, cotton candy, vanilla flavors.

Non-limiting examples of other food ingredients include flavors, acidulants, mineral, organic and amino acids, coloring agents, bulking agents, modified starches, gums, texturizers, preservatives, antioxidants, emulsifiers, stabilisers, thickeners, gelling agents.

The following examples illustrate various embodiments of the invention. It will be understood that the invention is not limited to the materials, proportions, conditions and procedures set forth in the examples, which are only illustrative.

### EXAMPLE 1 Preparation of stevia composition

1g of rebaudioside *A* produced by PureCircle Sdn. Bhd. (Malaysia), containing, 98.1% rebaudioside A, 0.3% stevioside, 0.2 rebaudioside C, 0.2% rebaudioside F, 0.4% rebaudioside B and 0.6% rebaudioside D was dissolved in 10mL 0.1M phosphate buffer (pH 7.0) and about 0.1mL of commercial lactase preparation - Maxilact® obtained from DSM Food Specialties B.V. (Netherlands), was added. The mixture was incubated at 37°C for 36 hours. Then the mixture was boiled at 100°C for 15 min and filtered through the layer of activated carbon. The filtrate temperature was adjusted to 20°C and the pH was adjusted to pH 4.0 with ortho-phosphoric acid. The solution was held under moderate agitation conditions for 4 hours and a precipitate was formed. The precipitate was filtered and washed on the filter with 2000mL of water. The washed crystals were dried under vacuum to yield 0.9g material containing about 80% reb *A* and 20% reb *B.* The water solubility (at 25°C) of obtained material was about 0.16% (w/v).

### EXAMPLE 2 Preparation of biocatalyst

A strain of *Kluyveromyces lactis* St-3010 (PureCirclc Sdn Bhd Collection of Industrial Microorganisms - Malaysia) was inoculated in 8 liters of sterilized culture medium containing 1.5% lactose, 0.5% rebaudioside A, 1.0% peptone, 0.5% yeast extract, and 0.5% (NH₄)₂HPO₄ (pH 6.0) at 28°C for 48 hrs with continuous aeration (8 L/min) and agitation (300rpm). The obtained culture broth was centrifuged at 4,500g for 20 min on a Sigma 3-16 K (Germany) centrifuge to separate the cells. The cells were subsequently washed with deionized water to obtain 250mL of biocatalyst.

### EXAMPLE 3 Preparation of stevia composition

1g of rebaudioside *A* produced by PureCircle Sdn. Bhd. (Malaysia), containing, 98.1% rebaudioside A, 0.3% stevioside, 0.2 rebaudioside C, 0.2% rebaudioside F, 0.4% rebaudioside B and 0.6% rebaudioside D was dissolved in 10mL 0.1M phosphate buffer (pH 7.0) and about 0.5mL of biocatalyst prepared according to EXAMPLE 2 was added. The mixture was incubated at 37°C for 36 hours. Then the mixture was boiled at 100°C for 15 min and filtered through the layer of activated carbon. The filtrate temperature was adjusted to 20°C and the pH was adjusted to pH 4.0 with ortho-phosphoric acid. The solution was held under moderate agitation conditions for 4 hours and a precipitate was formed. The precipitate was filtered and washed on the filter with 2000mL of water. The washed crystals were dried under vacuum to yield about 0.79g material containing about 2% reb *A* and about 98% reb *B.* The water solubility (at 25°C) of obtained material was about 0.1% (w/v).

### EXAMPLE 4 Preparation of soluble stevia composition

50 g material prepared according to EXAMPLE 1 was mixed with 50g of water and incubated in thermostatted oil bath. The temperature was increased at 1°C per minute to 121°C. The mixture was maintained at 121°C for 1 hour and then the temperature was decreased to room temperature (25°C) at 1°C per minute. The solution was dried using YC-015 laboratory spray drier (Shanghai Pilotech Instrument & Equipment Co. Ltd., China) operating at 175°C inlet and 100°C outlet temperature. About 45g of an amorphous powder was obtained with about 25% (w/v) solubility in water (at 25°C).

### EXAMPLE 5 Preparation of soluble stevia composition

42 g of reb *A* produced by PureCircle Sdn. Bhd. (Malaysia) with purity of 99.2% (dry basis) and 8 g of reb *B* prepared according to EXAMPLE 3 were mixed with 50g of water and incubated in thermostatted oil bath. The temperature was increased at 1°C per minute to 121°C. The mixture was maintained at 121°C for 1 hour and then the temperature was decreased to room temperature (25°C) at 1°C per minute. The solution was dried using YC-015 laboratory spray drier (Shanghai Pilotech Instrument & Equipment Co. Ltd., China) operating at 175°C inlet and 100°C outlet temperature. About 47g of an amorphous powder was obtained with about 1.5% (w/v) solubility in water (at 25°C).

### EXAMPLE 6 Low-calorie orange juice drink

Orange concentrate (35%), citric acid (0.35%), ascorbic acid (0.05%), orange red color (0.01%), orange flavor (0.20%), and 0.05% stevia composition, were blended and dissolved completely in water (up to 100%) and pasteurized. The stevia composition was selected from a commercial stevia extract (containing stevioside 26%, rebaudioside *A* 55%, and 16% of other glycosides), a commercial rebaudioside *A* (containing 98.2% reb *A*) or material obtained according to EXAMPLE 5.

The sensory evaluations of the samples are summarized in Table 1. The data shows that the best results can be obtained by using the composition obtained according to EXAMPLE 5. Particularly the drinks prepared with said composition exhibited a rounded and complete flavor profile and mouthfeel.

**Table 1**

| Evaluation of orange juice drink samples | | | |
|---|---|---|---|
| Sample | Comments | | |
| | Flavor | Aftertaste | Mouthfeel |
| Stevia Extract | Sweet, licorice notes | Bitterness and aftertaste | Not acceptable |
| Reb *A* | Sweet, slight licorice notes | Slight bitterness and aftertaste | Not acceptable |
| EXAMPLE 5 | High quality sweetness, pleasant taste similar to sucrose, rounded and balanced flavor | Clean, no bitterness and no aftertaste | Full |

The same method can be used to prepare juices and juice drinks from other fruits, such as apples, lemons, apricots, cherries, pineapples, mangoes, etc.

### EXAMPLE 7 Zero-calorie carbonated beverage

Carbonated beverages according to the formulas presented in Table 2 were prepared.

**Table 2**

| Carbonated Beverage Formulas | | | |
|---|---|---|---|
| Ingredients | Quantity, % | | |
| | Stevia Extract | Reb *A* | EXAMPLE 5 |
| Cola flavor | 0.340 | 0.340 | 0.340 |
| ortho-Phosphoric acid | 0.100 | 0.100 | 0.100 |
| Sodium citrate | 0.310 | 0.310 | 0.310 |
| Sodium benzoate | 0.018 | 0.018 | 0.018 |
| Citric acid | 0.018 | 0.018 | 0.018 |
| Stevia composition | 0.050 | 0.050 | 0.050 |
| Carbonated water | to 100 | to 100 | to 100 |

The sensory properties were evaluated by 20 panelists. The results are summarized in Table 3.

**Table 3**

| Evaluation of zero-calorie carbonated beverage samples | | | |
|---|---|---|---|
| Taste attribute | Number of panelists detected the attribute | | |
| | Stevia Extract | Reb *A* | EXAMPLE 5 |
| Bitter taste | 15 | 10 | 0 |
| Astringent taste | 16 | 9 | 0 |
| Aftertaste | 14 | 12 | 0 |

| Comments | | | |
|---|---|---|---|
| Quality of sweet taste | Bitter aftertaste (15 of 20) | Bitter aftertaste (10 of 20) | Clean (20 of 20) |
| Overall evaluation | Satisfactory (1 of 20) | Satisfactory (5 of 20) | Satisfactory (20 of 20) |

The above results show that the beverages prepared using the composition obtained according to EXAMPLE 5 possessed the best organoleptic characteristics.

### EXAMPLE 8 Diet cookies

Flour (50.0%), margarine (30.0%) fructose (10.0%), maltitol (8.0%), whole milk (1.0%), salt (0.2%), baking powder (0.15%), vanillin (0.1%) and different stevia compositions (0.03%) were kneaded well in dough-mixing machine. The obtained dough was molded and baked in oven at 200°C for 15 minutes. The stevia compositions were selected from a commercial stevia extract (containing stevioside 26%, rebaudioside A 55%, and 16% of other glycosides), a commercial rebaudioside *A* (containing 98.2% reb *A*) and material obtained according to EXAMPLE 5.

The sensory properties were evaluated by 20 panelists. The best results were obtained in samples containing the composition obtained according to EXAMPLE 5. The panelists noted a rounded and complete flavor profile and mouthfeel.

### EXAMPLE 9 Yoghurt

Different stevia compositions (0.03%) and sucrose (4%) were dissolved in low fat milk. The stevia compositions were selected from a commercial stevia extract (containing stevioside 26%, rebaudioside A 55%, and 16% of other glycosides), a commercial rebaudioside A (containing 98.2% reb *A*) and the material obtained according to EXAMPLE 5. After pasteurizing at 82°C for 20 minutes, the milk was cooled to 37°C. A starter culture (3%) was added and the mixture was incubated at 37°C for 6 hours then at 5°C for 12 hours.

The sensory properties were evaluated by 20 panelists. The best results were obtained in samples containing the composition obtained according to EXAMPLE 5. The panelists noted a rounded and complete flavor profile and mouthfeel.

## Claims

1. A process for producing a stevia composition comprising rebaudioside *B*, comprising the steps of:
providing a stevia sweetener comprising rebaudioside *A*;
providing a biocatalyst capable of hydrolyzing *β*-glucosyl ester bonds in the stevia sweetener, wherein the biocatalyst is lactase;
dissolving the stevia sweetener and adding the biocatalyst to make a reaction mixture;
incubating the reaction mixture for about 12 to 48 hours to at least partially hydrolyze *β*-glucosyl ester bonds in the stevia sweetener;
cooling the mixture to about 10-30°C and adjusting the pH with acid to about pH 3.0-4.0;
incubating the mixture at low temperature to obtain a precipitate;
separating the precipitate and washing the precipitate with water; and
drying the washed precipitate to obtain the stevia composition.

2. The process of claim 1 further comprising the steps of:
suspending the stevia composition in water and incubating at an elevated temperature for about 1-3 hours; and
separating the stevia composition from the water and drying the stevia composition to obtain a purified stevia composition comprising rebaudioside *B* having a purity of greater than about 99%.

3. The process of claim 1 or 2 further comprising the steps of:
suspending the stevia composition in water to form a suspension;
increasing the temperature of the suspension by a gradient heating method;
holding the suspension at an elevated temperature;
decreasing the temperature of the suspension by a gradient cooling method to obtain a high stability and high concentration stevia composition solution; and
spray drying the high stability and high concentration stevia composition solution to provide a highly soluble stevia composition.

## Patentansprüche

1. Verfahren zur Herstelllung einer Stevia-Zusammensetzung, umfassend Rebaudiosid B, das die Schritte umfasst:
Bereitstellen eines Steviasüßstoffs, umfassend Rebaudiosid A;
Bereitstellen eines Biokatalysatoren der ß-Glukosylesterbindungen in dem Steviasüßstoff hydrolisieren kann, wobei der Biokatalysator Laktase ist;
Auflösen des Steviasüßstoffs und Zugeben des Biokatalysatoren, um ein Rekationsgemisch zu erstellen;
Inkubation des Reaktionsgemisches für etwa 12 bis 48 Stunden, um ß-Glukoseesterbindungen in dem Steviasüßstoff zumindest teilweise zu hydrolisieren;
Kühlen des Gemisches auf etwa 10-30°C und Einstellen des pHs mit Säure auf etwa pH 3,0-4,0;
Inkubation des Gemisches bei niedriger Temperatur, um ein Präzipitat zu erhalten;
Abtrennen des Präzipitats und Waschen des Präzipitats mit Wasser; und
Trocknen des gewaschenen Präzipitats, um eine Stevia-Zusammensetzung zu erhalten.

2. Verfahren nach Anspruch 1, weiterhin umfassend die Schritte:
Suspendieren der Stevia-Zusammensetzung in Wasser und Inkubation bei einer erhöhten Temperatur für etwa 1-3 Stunden; und
Abtrennen der Stevia-Zusammensetzung vom Wasser und Trocknen der Stevia-Zusammensetzung, um eine aufgereinigte Stevia-Zusammensetzung zu erhalten, die Rebaudiosid B mit einer Reinheit von mehr als 99% umfasst.

3. Verfahren nach Anspruch 1 oder 2, weiterhin umfassend die Schritte:
Auflösen der Stevia-Zusammensetzung in Wasser um eine Suspension zu bilden;
Erhöhen der Temperatur der Suspension mittels eines Gradientenerhitzungsverfahrens;
Halten der Suspension bei einer erhöhten Temperatur;
Erniedrigen der Temperatur der Suspension mittels eines Gradientenkühlungsverfahrens, um eine hochstabile und hochkonzentrierte Stevia-Zusammensetzungslösung zu erhalten; und
Sprühtrocken der hochstabilen und hochkonzentrierten Stevia-Zusammensetzungslösung, um eine hochlösliche Stevia-Zusammensetzung bereitzustellen.

## Revendications

1. Procédé de production d'une composition de stevia comprenant du rébaudioside B, comprenant les étapes de :
Fournir un édulcorant de stevia comprenant du rébaudioside A ;
Fournir un biocatalyseur capable d'hydrolyser des liaisons β-glucosyl ester dans l'édulcorant de stevia, dans laquelle le biocatalyseur est une lactase ;
Dissoudre l'édulcorant de stevia et ajouter le biocatalyseur pour préparer un mélange réactionnel ;
Incuber le mélange réactionnel pendant environ 12 à 48 heures pour hydrolyser au moins partiellement les liaisons β-glucosyl ester dans l'édulcorant de stevia ;
Refroidir le mélange à environ 10-30°C et ajuster le pH avec un acide à un pH d'environ 3,0-4,0 ;
Incuber le mélange à basse température pour obtenir un précipité ;
Séparer le précipité et laver le précipité avec de l'eau ; et
Sécher le précipité lavé pour obtenir la composition de stevia.

2. Procédé selon la revendication 1, comprenant en outre les étapes de :
Suspendre la composition de stevia dans l'eau et incuber à une température élevée pendant environ 1-3 heures ; et
Séparer la composition de stevia de l'eau et sécher la composition de stévia pour obtenir une composition de stevia purifiée comprenant du rébaudioside B ayant une pureté plus élevée que 99% environ.

3. Procédé selon la revendication 1 ou 2, comprenant en outre les étapes de :
Suspendre la composition de stevia dans l'eau pour former une suspension ;
Augmenter la température de la suspension par une méthode de gradient de chauffage ;
Maintenir la suspension à une température élevée ;
Diminuer la température de la suspension par une méthode de gradient de refroidissement pour obtenir une solution de composition de stevia à concentration élevée et à stabilité élevée ; et
Sécher par atomisation la solution de composition de stevia à concentration élevée et à stabilité élevée pour fournir une composition de stevia hautement soluble.
